(19) Europäisches Patentamt
European Patent Office
Office européen des brevets

(11) Publication number: 0 017 390 A2

# (12) EUROPEAN PATENT APPLICATION

(21) Application number: 80300845.7

(22) Date of filing: 19.03.80

(51) Int. Cl.³: C 07 D 513/04
C 07 D 207/16, A 61 K 31/54
//(C07D513/04, 279/00, 209/00),
(C07D513/04, 281/00, 209/00)

(30) Priority: 23.03.79 US 23105
01.08.79 GB 7926808
09.10.79 US 83052

(43) Date of publication of application:
15.10.80 Bulletin 80/21

(84) Designated Contracting States:
AT BE CH DE FR GB IT LU NL SE

(71) Applicant: AMERICAN HOME PRODUCTS CORPORATION
685, Third Avenue
New York, New York 10017(US)

(72) Inventor: Kim, Dong Han
109 Oakford Circle
Wayne Pennsylvania 19087(US)

(74) Representative: Porter, Graham Ronald et al,
C/O John Wyeth & Brother Limited Huntercombe Lane
South Taplow
Maidenhead Berkshire, SL6 0PH(GB)

(54) **Bicyclic oxo-thiolactones, pharmaceutical compositions containing them, processes for their preparation and compounds useful as intermediates for their preparation.**

(57) Disclosed herein are bicyclic oxo-thiolactones of the formula:

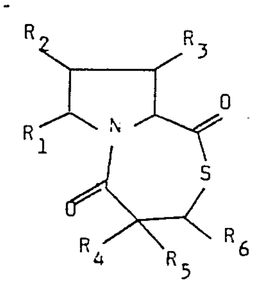
(Ia)

or

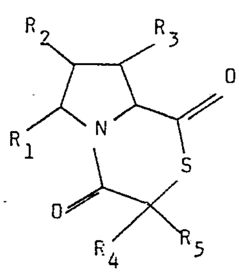
(Ib)

in which $R_1$ is hydrogen or lower alkyl; $R_2$ and $R_3$ are, independently, hydrogen, hydroxy, halogen, lower alkyl, lower alkoxy, or aryl; and $R_4$, $R_5$ and $R_6$ are independently, hydrogen, lower alkyl, lower hydroxyalkyl, aryl or aryl-alkyl, except that $R_4$ and $R_5$ may not both be aryl

These compounds act as inhibitors or angiotension converting enzyme and are useful as agents for the treatment of hypertension in mammals. The compounds are stable against oxidation of the sulphur.

Also disclosed are methods of preparation of such compounds and novel intermediates having the formula

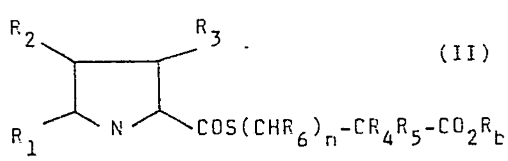
(II)

where Ra is hydrogen or an amino protecting group, $R_b$ is hydrogen or a carboxylic acid protecting group, n is 1 or 0 and their salts.

EP 0 017 390 A2

Croydon Printing Company Ltd.

AHP-7449-EPC 0017390



BICYCLIC OXO-THIOLACTONES, PHARMACEUTICAL COMPOSITIONS CONTAINING THEM, PROCESSES FOR THEIR PREPARATION AND COMPOUNDS USEFUL AS INTERMEDIATES FOR THEIR PREPARATION.

American Home Products Corporation, a corporation organised and existing under the laws of the State of Delaware, United States of America, of 685 Third Avenue, New York 10017, New York, United States of America.

This invention concerns bicyclic oxo-thiolactones of the formula:-

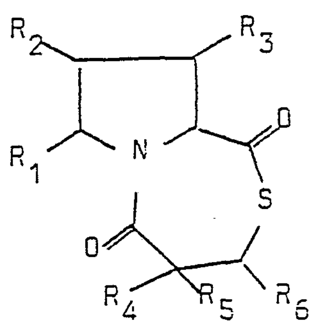

Ia

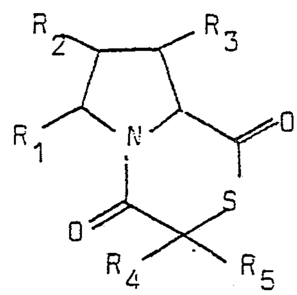

Ib

in which $R_1$ is hydrogen or lower alkyl; $R_2$ and $R_3$ are, independently, hydrogen, hydroxy, halogen, lower alkyl, lower alkoxy, or aryl; and $R_4$, $R_5$ and $R_6$ are, independently, hydrogen, lower alkyl, lower hydroxyalkyl, aryl or arylalkyl, except that $R_4$ and $R_5$ may not both be aryl.

The compounds of the invention act as inhibitors of angiotensin converting enzyme and are useful as agents for the treatment of hypertension and for the study of the renin-angiotensin-aldosterone system of warm-blooded animals. Also within the scope of the invention are the compounds as pharmaceuticals, pharmaceutical compositions containing the compounds, methods of treatment of hypertension utilizing the compound of the invention, processes for the preparation of the compounds, intermediate compounds and processes for the preparation of the intermediates.

As used herein "lower alkyl" and "lower alkoxy" refer to groups having up to 4 carbon atoms. "Aryl" refers to phenyl or phenyl substituted by a halogen, lower alkyl, lower alkoxy, or hydroxy group. "Arylalkyl" refers to benzyl or benzyl substituted as above for phenyl. Halogen refers to chlorine, bromine and fluorine.

In pharmacological research on hypertension, recent attention has focused on the study of the renin-angiotensin-aldosterone system, and, in particular, on the development of an effective anti-hypertensive agent which would, theoretically, achieve its result by inhibiting the action of angiotensin converting enzyme in converting angiotensin I to angiotensin II. The inhibition of the production of angiotensin II became important because of the discoveries that angiotensin II is the most potent pressor agent (vasoconstrictor) present in the mammalian body and in addition, stimulates the adrenal cortex to release aldosterone, thereby causing excessive sodium retention and fluid retention, contributing further to the hypertensive state. Thus, inhibiting the conversion of angiotensin I to angiotensin II is believed to work directly on the primary biochemical mechanisms creating increased blood pressures. For a description of these mechanisms and of the mammalian renin-angiotensin-aldosterone system, see Hypertension, Genest et al., ed., Chapters 6.1, 6.2, 7.1, 7.2 and 7.3 (McGraw-Hill, 1977) and John H. Laragh, "The Renin System in High Blood Pressure, From Disbelief to Reality: Converting-Enzyme Blockade for Analysis and Treatment, "Prog. in Cardio. Vasc. Disease, XXI, No. 3, 159-166 (November, 1978).

An extensive list of angiotensin converting enzyme inhibitors is set forth in Suzanne Oparil's article entitled "Angiotensin I Converting Enzyme and Inhibitors" in Genest et al., supra, Chapter 6.3, at pp. 159-161. These inhibitors are summarised in Table I, p 161, thereof and include chelating agents, sulphonylating

agents, heavy metal ions, sulphhydryl binding reagents, and various peptides. The polypeptides described therein as angiotensin converting enzyme inhibitors include hormones, such as bradykinin; products of substrate digestion such as His-Leu, Phe-Arg and Arg-Pro-Pro; and various snake venom polypeptide extracts. Two of the most potent and most studied inhibitors are the Bothrops jararaca snake venom extract, the pentapeptide (Pyr-Lys-Trp-Ala-Pro), also referred to as $BPP_{5a}$, and the nonapeptide (Pyr-Trp-Pro-Arg-Pro-Gln-Ile-Pro-Pro), also referred to as $BPP_{9a}$. (BPP stands for Bradykinin Potentiating Peptide). $BPP_{9a}$ has been shown to be an effective anti-hypertensive agent in clinical studies on humans with certain forms of hypertension. However, $BPP_{9a}$ is not orally active as an anti-hypertensive agent. For a summary of the clinical aspects of $BPP_{9a}$ see Genest et al., supra, Chapter 6.3, pp. 163-4.

More recently, a series of proline derivatives has been found to be significantly more potent as inhibitors of angiotensin converting enzyme and as anti-hypertensive agents than $BPP_{9a}$. Of these proline derivatives, D-3-mercapto-2-methylpropanoyl-L-proline has been reported to be the most effective, including being effective when administered orally. These proline and mercapto-proline derivatives and various pharmacological test results thereon are described in Cushman et al., "Design of New Anti-hypertensive Drugs: Potent and Specific Inhibitors of Angiotensin Converting Enzyme", Prog. in Cardio. Diseases, Vol. XXI, No. 3 (Nov./Dec., 1978), and in United States Patent Nos. 4,046,889 and 4,105,776, both to Ondetti and Cushman.

The generic description of the compounds of the invention is given above (Formulae 1a and 1b). Preferred compounds of the invention include those in which $R_2$ and $R_3$ are hydrogen; $R_1$, $R_2$ and $R_3$ are hydrogen; $R_1$, $R_5$ and $R_6$ are hydrogen; $R_2$, $R_3$, $R_5$ and $R_6$ are hydrogen;

$R_1$, $R_2$, $R_3$, $R_5$ and $R_6$ are hydrogen; $R_1$, $R_2$, $R_3$, $R_5$ and $R_6$ are hydrogen and $R_4$ is hydrogen or lower alkyl (particularly preferred are the 4S, 9aS or 3S,8aS and 4R,9aS or 3R,8aS stereoisomeric forms of these compounds); and $R_1$, $R_2$, $R_3$ and $R_5$ are hydrogen and $R_4$ and $R_6$ are hydrogen or lower alkyl.

Another subgeneric aspect of the invention is those compounds of the formula:

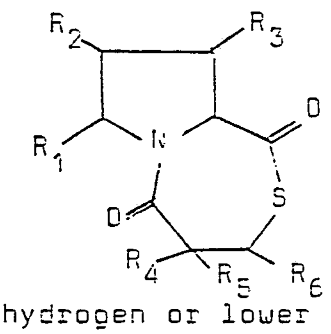

(Ia)

in which $R_1$ is hydrogen or lower alkyl; $R_2$ and $R_3$ are, independently, hydrogen, hydroxy, halogen, lower alkyl, lower alkoxy or aryl; and $R_4$, $R_5$ and $R_6$ are, independently, hydrogen, lower alkyl, lower hydroxyalkyl, aryl or arylalkyl, except that $R_4$ and $R_5$ may not both be aryl. Preferred compounds of this aspect of the invention include those in which $R_2$ and $R_3$ are hydrogen; $R_1$, $R_2$ and $R_3$ are hydrogen; $R_1$, $R_5$ and $R_6$ are hydrogen; $R_2$, $R_3$, $R_5$ and $R_6$ are hydrogen; $R_1$, $R_2$, $R_3$, $R_5$ and $R_6$ are hydrogen; $R_1$, $R_2$, $R_3$, $R_5$ and $R_6$ are hydrogen and $R_4$ is hydrogen or lower alkyl (particularly preferred are the 4S,9aS and 4R,9aS stereoisomeric forms of these compounds); and $R_1$, $R_2$, $R_3$ and $R_5$ are hydrogen and $R_4$ and $R_6$ are hydrogen or lower alkyl.

Another subgeneric aspect of the invention is those compounds of the formula:

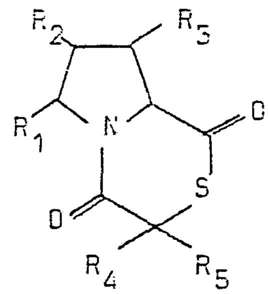

(Ib)

in which $R_1$ is hydrogen or lower alkyl; $R_2$ and $R_3$ are, independently, hydrogen, hydroxy, halogen, lower alkyl, lower alkoxy, or aryl; and $R_4$ and $R_5$ are, independently, hydrogen, lower alkyl, lower hydroxyalkyl, aryl or arylalkyl, except that $R_4$ and $R_5$ may not both be aryl. Preferred compounds of this aspect of the invention include those in which $R_2$ and $R_3$ are hydrogen; $R_1$, $R_2$ and $R_3$ are hydrogen; $R_1$ and $R_5$ are hydrogen; $R_2$, $R_3$, and $R_5$ are hydrogen; $R_1$, $R_2$, $R_3$ and $R_5$ are hydrogen and $R_4$ is hydrogen or lower alkyl (particularly preferred are the 3S,8aS and 3R,8aS stereoisomeric forms of these compounds).

As previously noted, this invention also includes methods of treatment of hypertension in warm-blooded animals utilizing hypotensively effective amounts of the compounds of the invention. The generic and subgeneric aspects of this part of the invention encompass the same compounds and groups of compounds described above with respect to the compound portion of the invention.

The compounds of formulae Ia and Ib are stable against oxidation and polymerization of the sulphur atom.

The intermediate compounds for which patent protection is sought have the general structural formula:

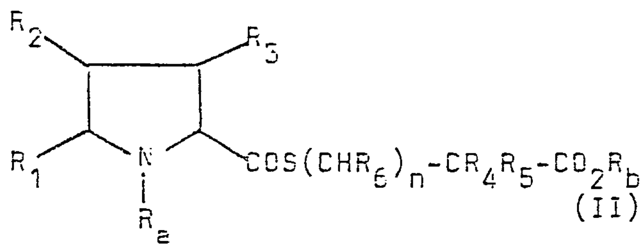

(II)

in which $R_1$, $R_2$, $R_3$, $R_4$, $R_5$ and $R_6$ are the same as defined above; n is 1 or 0; $R_a$ is hydrogen or an amino protecting group; and $R_b$ is hydrogen, a carboxylic acid protecting group or a group having the formula

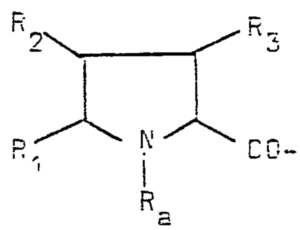

(III)

wherein $R_a$, $R_1$, $R_2$ and $R_3$ are as defined above, and their salts including acic addition salts.

The intermediate compounds of the invention include the group having the general formula

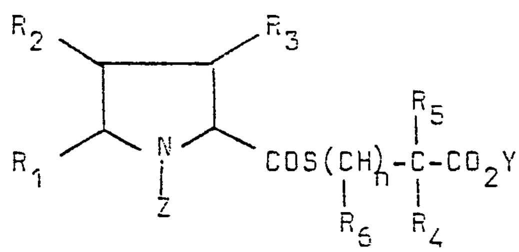

(IV)

in which $R_1$, $R_2$, $R_3$, $R_4$, $R_5$ and $R_6$ are the same as defined above; n is 1 or 0; Y is a carboxylic acid protecting group; and Z is an amino protecting group.

A further group of intermediate compounds of the invention have the following structural formula :

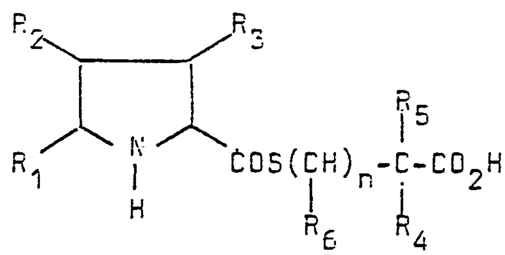

(V)

in which $R_1$, $R_2$, $R_3$, $R_4$, $R_5$ and $R_6$ are the same as defined above; and n is 1 or 0.

It will be apparent to one skilled in the art that these intermediate compounds may be formed as salts in the deprotection processes and such salts and compounds or other salts of the compounds may be readily interconverted.

The invention provides a process for the preparation of a compound having the general formula

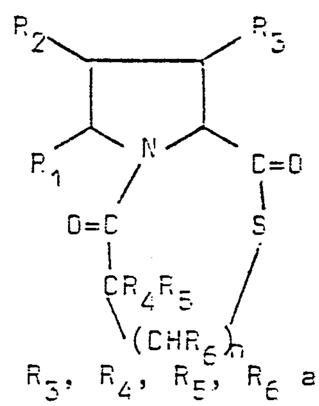

(I)

(wherein $R_1$, $R_2$, $R_3$, $R_4$, $R_5$, $R_6$ and n are as defined above) which comprises

(A) cyclising a compound having the general formula

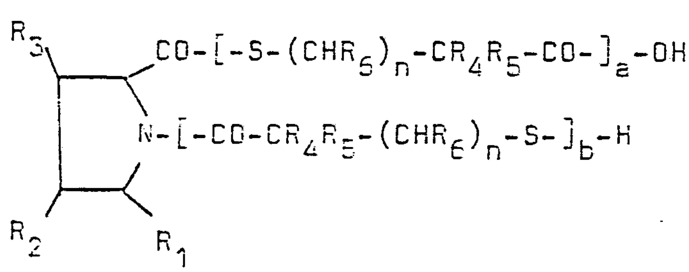

(VI)

(wherein $R_1$, $R_2$, $R_3$, $R_4$, $R_5$, $R_6$ and n are as defined above, one of a and b represents 0 and the other one of

a and b represents 1) or a reactive derivative thereof;

or

(E) cyclising a compound having the formula

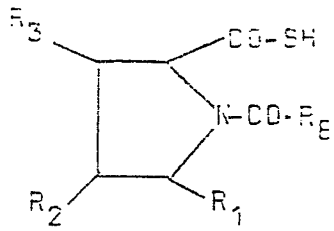

(VII)

(wherein $R_1$, $R_2$, $R_3$, are as defined above subject to the proviso that neither of $R_2$ and $R_3$ is halogen and $R_8$ represents a group having the formula $-CR_4R_5-(CHR_6)_n-R_7$ or $-CR_4=CHR_6$ where $R_4$, $R_5$, $R_6$, and n are as defined above subject to the proviso that none of $R_4$, $R_5$ and $R_6$ is halophenyl and $R_7$ is a leaving group or atom) or a salt thereof.

When n is 1, the compounds produced are according to formula Ia above. When n is 0, the compounds produced by the process are according to formula Ib above.

The compounds having formula VI where a is 1 and b is 0 are novel compounds provided by the invention (see formula V). They can be prepared by methods described hereinafter. They can be cyclised by means of any of many coupling reagents used in peptide synthesis, for example, dicyclohexylcarbondiimide. Addition of a small amount of 4-dimethylamino-pyridine as catalyst is recommended. Alternatively the compounds having formula V may be used in the form of their reactive derivatives. Such reactive derivatives may be prepared in manner known per se. As examples there may be mentioned the derivatives with an activated carboxyl group, for instance, acyl halides, acid anhydrides and active esters. By way of example, the compound having the formula V is converted into its acid chloride hydrochloride having the formula

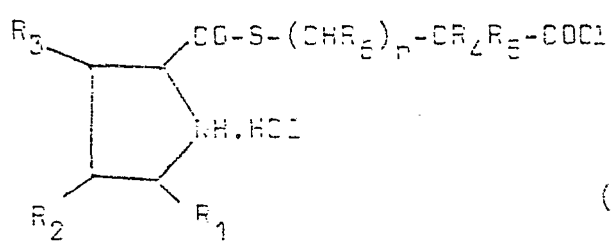

(VIII)

(wherein $R_1$, $R_2$, $R_3$, $R_4$, $R_5$, $R_6$ and n are as defined above) by thionyl chloride, phosphorus oxychloride or phosphorus pentachloride. Treatment of the acid chloride hydrochloride with a base under such conditions that the thioester function is not hydrolysed converts the amine into its free base form which can be acylated by the acid chloride function so as to effect cyclisation.

The compounds having formula VI where a is 0 and b is 1 are 1-(mercaptoacyl)proline derivatives which can be prepared as described in United States Patents Nos. 4,046,889 and 4,105,776 to Ondetti et al. The cyclisation of the 1-(mercaptoacyl)proline derivates can be carried out as hereinafter described, in particular by means of a coupling agent. Alternatively, the reactive derivatives of 1-(mercaptoacyl)prolines, for example, derivatives with activated carboxyl groups, can be prepared in known manners and used to form the oxo-thiolactone of formula I by cyclisation.

In formula VII $R^7$ preferably represents a halogen atom or a sulphonate ester such as tosylate or mesylate. The compounds having the formula VII and their salts can be prepared in known manner. For instance, a carboxy-protected proline having the formula

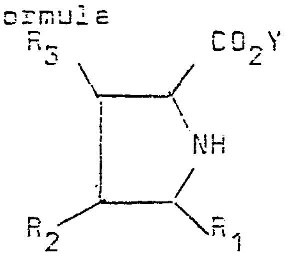

(IX)

(where $R_1$, $R_2$ and $R_3$ are as defined above and Y is a carboxylic acid protecting group) is acylated to introduce the acyl group having the formula

$$-CO-R_6 \qquad (X)$$

wherein $R_8$ is as defined above, the carboxylic acid protecting group is removed in known manner, the resultant carboxylic acid is converted into its acid chloride by reaction with phosphorus pentachloride or thionyl chloride and the acid chloride is reacted with hydrogen sulphide to form the desired thioacid. The thioacid of formula VII where $R_8$ is a group having the formula $-CR_4R_5-(CHR_6)_n-R_7$ or its salt can be cyclised by standard procedures for the alkylation of thioacids. The thioacids of formula VII where $R_8$ is $-CR_4=CHR_6$ or a salt thereof, can be cyclised by an internal Michael Addition by standard procedures to obtain a compound having formula Ia where $R_5$ is hydrogen. [See, for instance, Methoden Der Organischen Chemie (Houben-Weyl), Volume IX, pages 749-752, published by Georg Thieme Verlag, Stuttgart (1955)].

The invention also provides a process for the preparation of a compound having the general formula II or a salt thereof, which comprises acylation of a compound having the general formula

$$HS-(CHR_6)_n-CR_4R_5-CO_2H \qquad (XI)$$

(wherein $R_4$, $R_5$, $R_6$, and n are as defined above) or a derivative thereof with an acid having the general formula

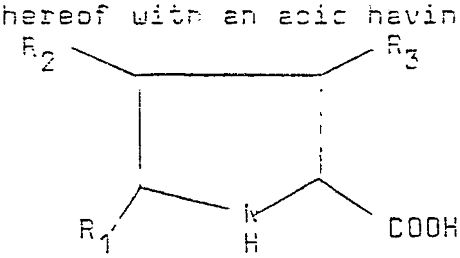

(XII)

(wherein $R_1$, $R_2$, and $R_3$ are as defined above) or a derivative thereof to form a thioester.

It will be appreciated by the reader that formulae XI and XII present two sites liable to be acylated, the mercaptan group in formula XI and the nitrogen atom in the formula XII, and also two carboxyl groups capable of effecting acylation. Undesired acylation reactions can be avoided by standard procedures of peptide chemistry. In particular groups that are not intended to react may

be protected in known manner and groups that are intended to react may be activated in known manner.

Undesired acylation at the nitrogen atom in formula XII can be avoided by using the N-carboxyanhydride derivative having formula

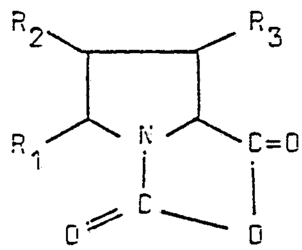

(XIII)

(wherein $R_1$, $R_2$ and $R_3$ are as defined above) as the acylating agent. The N-carboxyanhydride can be prepared by procedures well known in the art. A preferred method involves reaction of the amino acid of formula XII with phosgene under anhydrous conditions in a reaction inert organic solvent, for instance p-dioxane. Alternatively, such undesired acylation may be avoided by using an N-protected acylating agent. Protection may be afforded by protonation. For instance the acid chloride hydrochloride derivative having the formula

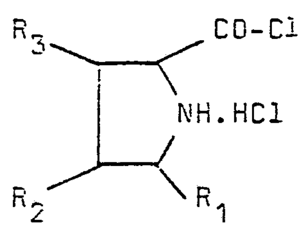

(XIV)

(wherein $R_1$, $R_2$ and $R_3$ are as defined above) may be used as acylating agent. Alternatively, protection may be afforded by means of a protecting group. For example, as acylating agent one may use the N-(t-butyloxycarbonyl) derivative of the compound having formula XII or its acid chloride.

One may avoid undesired acylation as a result of the carboxy group in formula XI by (a) activation of the carboxy group in formula XII, (b) protection of the carboxy group in formula XI or both (b) and (a). Method (b), protection of the carboxy group of the thiol-carboxylic acid, is discussed in detail below. Method (a), activation of the carboxy group, can be illustrated by use of an active ester, acyl halide or N-carboxyanhydride derivative as the acylating agent. For instance, a compound having the formula XIII or XIV can be used as acylating agent.

If the acylating agent involves an activated carboxy group whilst the carboxy group of the thiol-carboxylic acid (formula XI) is unprotected, one may form a mixed anhydride where $R_b$ is a group of formula III in formula II especially where excess acylating agent is used. Such a mixed anhydride may be converted to the carboxylic acid itself by hydrolysis under conditions where the thioester linkage is not cleaved. Alternatively, the mixed anhydride group may be retained if convenient for use in subsequent cyclisation to form a compound having formula I.

Where the carboxy group of formula XII is used as such rather than in the form of an activated derivative, the acylation may be effected by adding a coupling reagent, for instance, as described below.

The aforesaid process for the preparation of a compound having formula II or a salt thereof may further include any one or more of the following steps:-

(a) removal of a carboxylic acid protecting group and/or a secondary amine protecting group;

(b) hydrolysis of a compound where $R_b$ has formula III to form an acylmercapto carboxylic acid;
and

(c) conversion of a compound having formula II salt thereof or a salt into a compound having formula II.

The invention also provides a further process for the preparation of a compound having formula II or a salt thereof, which comprises reaction of a thioacid having the formula

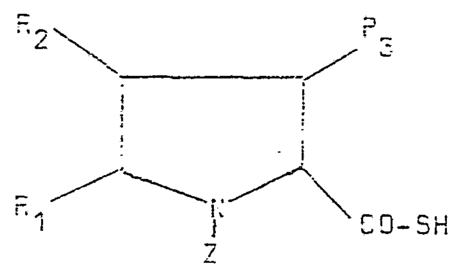

(XV)

(wherein Z, $R_1$, $R_2$ and $R_3$ are as defined above subject to the proviso that neither of $R_2$ and $R_3$ is halogen) or a salt thereof with a compound having the formula $R_a$-$CO_2R_b$(XVI) (wherein $R_b$ is as defined above and $R_a$ represents a group having the formula $-CR_4R_5-(CHR_6)_n-R_7$ or $-CR_4=CHR_6$ wherein $R_4$, $R_5$, $R_6$, $R_7$, $R_b$ and n are as defined above subject to the proviso that none of $R_4$, $R_5$ and $R_6$ is halophenyl) or a salt thereof. $R_7$ is preferably a halogen atom or sulphonate ester such as tosylate or mesylate. In order to prepare a compound having formula II where $R_a$ is hydrogen, the amino protecting group should be removed. Where $R_a$ represents a group having

the formula-$CR_4$=$CHR_6$, the product obtained is of formula II where n is 1 and $R_5$ is hydrogen. This process may be carried out in manner known for the alkylation of thioacids.

Where it is intended to prepare a compound of formula I in which $R_2$ or $R_3$ is hydroxy or $R_4$, $R_5$ or $R_6$ is lower hydroxyalkyl or hydroxyphenyl by a method involving acylation, it is recommended that the hydroxy group be protected prior to the acylation reaction and the protection removed subsequently. Thus the derivatives used of compounds having formula VI, XI and XII include cases where such a hydroxy group is protected by means of a hydroxy-protecting group. The preparation and removal of hydroxy-protecting groups is described in chapter 3 and 4 of McOmie, J.F.W.,ed.,Protective Groups in Organic Chemistry, Plenum Press(1973) at pages 95-182.

The following flow diagram illustrates a general method for preparing compounds having formulas Ia and Ib.

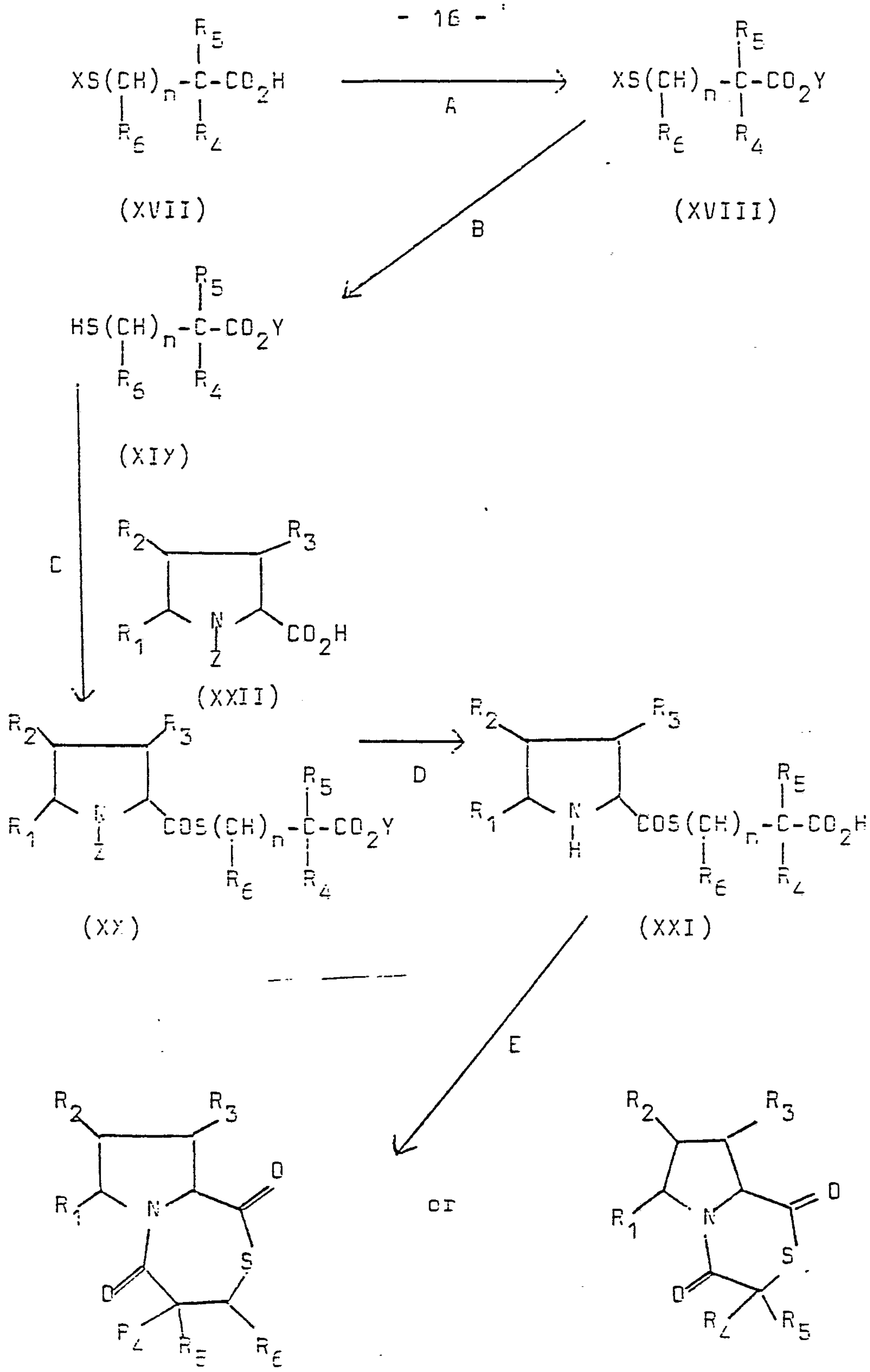


(XVII) (XVIII)

(XIY)

(XXII)

(XX) (XXI)

(Ia) or (Ib)

wherein $R_1$, $R_2$, $R_3$, $R_4$, $R_5$ and $R_6$ are as previously defined; n is 1 or 0; X is a thiol protecting group; Y is a carboxylic acid protecting group; and Z is a secondary amino protecting group.

In step A of the above reaction sequence, the carboxylic acid moiety of the (S-protected) thiol-carboxylic acid (XVII) starting material is treated in a conventional manner to form a protected ester (XVIII). In step B the thiol protecting group (X) is removed in a known manner to produce the thiol-carboxylic acid ester (XIX). In step C of this sequence the thiol-carboxylic acid ester (XIX) is reacted with the (N-protected) proline (XXII) to form the proline thioester (XX). In step D deprotection of both the secondary amine and the carboxylic acid (i.e. removal of protecting groups Z and Y) is preferably performed with one reagent to yield the unprotected intermediate (XXI). In step E the unprotected intermediate (XXI) is treated, preferably with a dehydrating agent, to cause the lactam formation, thereby forming the oxo-thiolactone ring of compounds Ia and Ib of the invention. These steps are preferably carried out in a nitrogen or other inert atmosphere.

The (protected) thiol-carboxylic acid (XVII) starting material may be a commercially available product or may be made from readily available reagents by methods well known to those skilled in the art of organic chemistry. X, the thiol-protecting group of XVII, may be a known sulphhydryl protecting group such as the acetyl, benzoyl, or substituted benzoyl (e.g. p-nitrobenzoyl) thioester groups; or a thioether formed with a benzyl group. The reagents and methods of forming such (S-protected) thiol-carboxylic acids XII are known to those skilled in the art. The thiol protecting group chosen should not be removable by such conditions that the carboxylic acid protecting group is also removed. Such thioester protecting groups may be

removed (deprotection of step B) by treatment with an ammonia-methanol solution, aqueous ammonia solution, or sodium methoxide in methanol solution. The preparation and removal of thioether and thioester protecting groups is described in Chapter 7 of McOmie, J.F.W., ed., Protective groups In Organic Chemistry, Plenum Press (1973) at pages 241-270 and 286-295, respectively. The acetyl protecting group is preferred and the methanolic ammonia solution is the preferred reagent for deprotection of said sulphhydryl group (step B).

The carboxylic acid protecting group, Y, may be a *t*-butyl group or a substituted benzyl group, such as a *p*-methoxybenzyl or a 2,4,6-trimethyl benzyl group; or a benzhydryl group. The *t*-butyl group is preferred. The removable carboxylic acid protecting group utilized, preferably, should be removable under the same conditions by which the secondary amino protecting group Z is removable, but should not be removable under the conditions used to remove the thiol protecting group X.

The esterification of the (S-protected) thiolcarboxylic acid (XVII) with a t-butyl group may be accomplished by reacting the compound XVII with t-butyl alcohol in an inert solvent, such as chloroform or dioxane, in the presence of a condensation agent such as dicyclohexylcarboxiimide; or by reacting the compound XVII with isobutylene in methylene chloride in the presence of an acid catalyst such as sulphuric acid. The latter method is preferred.

The benzyl and substituted benzyl esters may be formed by reacting the compound XVII with the appropriate benzyl alcohol in an inert solvent, such as benzene in the presence of an acid catalyst, such as sulphuric acid.

The t-butyl, benzyl and substituted benzyl groups are preferably removed using trifluoroacetic acid at from 0°C to room temperatures (25°C). Other methods of removing and forming the carboxylic acid protecting group Y are well known to those skilled in the organic

chemistry. Such methods and reagents, as well as other suitable carboxylic acid protecting groups, are described in McOmie, supra, Chapter 5, particularly pages 196-197 with respect to the t-butyl and benzyl or substituted benzyl esters and table 5.1.

As previously described, the sulphhydryl deprotection step B, may be accomplished by treating the double protected thiol-carboxylic acid (XVIII) with an ammonia-methanol solution, aqueous ammonia solution or sodium methoxide in methanol solution. The methanolic-ammonia solution is preferred. The time and temperature conditions are known to those skilled in the art. The use of other effective thiol protecting groups is described in McOmie, supra, Chapter 7.

With respect to preparation step C, suitable secondary amino protecting groups, Z, may be chosen from the urethane-type amino protecting groups such as those described in Table 4 of M.Bodanszky et al., Peptide Synthesis, pp. 35-7 (John Wiley & Sons, 1976). Preferred are such urethane type protecting groups which are removable by treatment with trifluoroacetic acid (TFA). Examples of such protecting groups are t-butyloxycarbonyl (Boc), 2-(p-biphenyl)isopropyloxycarbonyl (Bpoc) and benzyl or substituted benzyloxycarbonyl (e.g. p-methoxybenzyloxycarbonyl) groups. Boc may be introduced by reacting di-t-butyldicarbonate with the appropriate substituted proline compound. Other methods of introducing and of removing Boc and other amino protecting groups are well known in the peptide and penicillin arts and are described in, for example, Bodanszky et al., supra, pp. 18-49.

The formation of thioester (XX) from an N-protected proline (XXII) and mercaptoalkylcarboxylic acid which is protected at the carboxylic acid end (XIX) (process step C) can be accomplished by a coupling reagent used widely in the peptide synthesis, such as dicyclohexyl-

carbodiimide (DCC) or N-ethyl-N'-(3-dimethylaminopropyl)carbodiimide and related reagents. Examples of such reagents are found in pp. 116-121 "Peptide Synthesis" (2nd Ed.) by M. Bodanszky, Y.E.Klausner, and M.A.Ondetti, Wiley Interscience Publication, New York 1976. The presence of small amounts of 4-dimethylaminopyridine is used to accelerate the reaction and increase the yield. The preferred reagent for the reaction is DCC in the presence of 4-dimethylaminopyridine used at room temperature for several hours in a methylene chloride medium.

The same thioester can also be prepared by treatment of the reactants with diethyl phosphocyanidate (DEPC) or diphenyl phosphorazidate (DPPA) in dimethylformamide in the presence of triethylamine [Yamada et al. J. Org. Chem., 39, 3302 (1974)], or by means of carbonyldiimidazole or carbonyl-di-1,2,4-triazole [Gais, Angew. Chem. Int. Edit. Engl. 16, 244 (1977)].

As previously noted, removal of both the amino and carboxylic acid protecting groups (Y and Z) from the protected intermediate XX (i.e. step D) is preferably accomplished using the same reagent in one step. This is the reason for selecting protecting groups Y and Z which may be cleaved from the compounds XX under the same treatment, for example, using the preferred trifluoroacetic acid treatment. It will be obvious to those skilled in the art that this deprotection may be accomplished using more than one step or more than one cleaving agent, depending upon which Y and Z protecting groups were chosen for the particular synthesis. The methods and conditions for such deprotection reactions are known to those skilled in the art in the penicillin and peptide arts.

The cyclization of the unprotected intermediate XXI to form the oxo-thiolactone products (Ia or Ib), step E, can be achieved by any of many coupling reagents which

are used in the peptide synthesis. Dicyclohexylcarboxiimide (DCC) is a typical and preferred reagent for this peptide cyclization. An addition of a small amount of 4-dimethylaminopyridine which acts as a catalyst in the reaction is recommended. Other reagents and reaction conditions which are useful for peptide bond coupling reactions can be found in Bodanszky et al., supra, pp. 116-121.

An alternate method of producing the compounds of the invention involves making a thioester cyclization of an appropriately substituted 1-(mercaptoacyl)proline derivative. The reagents and reaction conditions which are useful for the preparation of the thioester XX in step C above can also be used for this alternate cyclization to form the oxo-thiolactones of the invention. The preparation of such 1-(mercaptoacyl)proline derivatives is described in United States Patent Nos. 4,046,889 and 4,105,776 to Ondetti et al.

Where any of $R_1$, $R_2$, $R_3$ or $R_5$ of formula Ia or Ib is other than hydrogen, the carbon atom to which it is attached will be assymetric carbon atom. Where $R_4$ and $R_5$ are different, then the carbon atom to which they are attached is assymetric. Additionally, the carbon atom designated 9a or 8a, which joins the two rings at the thio end, is also assymetric and may exist in an S or R form. The 9aS or 8aS form of the compounds corresponds to the L form of proline. Thus, the compounds of the invention (including intermediate compounds) exist in stereoisomeric forms or in racemic mixtures thereof, all of which are within the scope of the invention. Anyone skilled in the art will be able to obtain any particular stereoisomeric form by use of methods routine in the art. In general, the 9aS or 8aS isomer is the preferred isomeric form of the compounds of the invention.

The angiotensin converting enzyme (ACE) inhibitory property of the compounds is measured in vitro and in vivo. The in vitro assay utilizes rabbit lung extract and a specific tripeptide substrate, hippuryl-L-histidyl-L-leucine being preferred, and follows the method of Cushman et al., Biochem. Pharmacol., 20, 1637-1648, 1971.

The in vivo ACE inhibitory activity of the compounds is measured according to the procedure described in Rubin et al., J. Pharmacol. Exp. Ther., 204, 271-280, 1978, which utilizes the conscious normotensive rat as a subject. In this procedure the jugular vein and carotid artery cannulae are placed in an either-anesthetized, normotensive, male, Sprague-Dauley rat for injection or oral dosage of compounds and direct recording of systemic arterial pressure, respectively. The blood pressure responses to i.v. injection of angiotensin I (300 ng/kg), angiotensin II (100 ng/kg) and bradykinin (10 µg/kg) are recorded and compared with identical doses administered at various time intervals after oral dosing of a prospective angiotensin converting enzyme inhibitor. An angiotensin converting enzyme inhibitor would not be expected necessarily to lower arterial pressure in the normotensive rat, but would be expected to block angiotensin I pressor responses without grossly altering angiotensin II responses. Additionally, the vasodepressor response to bradykinin would be expected to be augmented since angiotensin converting enzyme is known to inactivate bradykinin normally.

When administered orally and intravenously according to this procedure, compounds of the invention showed an ability to inhibit angiotensin converting enzyme at doses of .1-10 mg/kg.

The anti-hypertensive effect of the compounds of the invention is measured in the spontaneously hypertensive rat. In this procedure systolic pressure of male spontaneously hypertensive rats is measured by an indirect

technique using the Decker Caudal Plethysmograph or other appropriate sensor. Groups usually consist of 4 or more rats. Drugs are usually administered orally. Pressures are usually read prior to drug administration and at 1.5, 4 and 24 hours thereafter. This schedule may be altered depending upon the behaviour of the drug.

This procedure measures the hypotensive effect of the subject compounds in a hypertensive subject using a single dose measuring the response over a 24 hour period. When administered orally according to this procedure, compounds of the invention produced a significant decrease in blood pressures in doses of 10-50 mg/kg. When used to treat hypertension in warm blooded animals doses of less than 15 mg/kg/day would be utilized, and preferably doses of 2-6 mg/kg/day. Such effective treatment doses would generally be administered in long term antihypertensive therapy. Angiotensin converting enzyme inhibitors when utilized as anti-hypertensive agents are most effective upon such extended administration and exhibit no significant side effects when administered at moderate or low doses. As noted earlier, the compounds of the invention exhibit a hypotensive (depressor) response only when administered to hypertensive subjects and would not be expected to lower blood pressures significantly in normotensive subjects.

The compounds of the invention may be administered orally, intravenously, intraperitoneally, intramuscularly, or subcutaneously. Oral administration is preferred.

When employed to lower blood pressures in hypertensive subjects the effective dosage of the compound being utilized for such treatment will vary according to the particular compound being employed, the severity and nature of condition being treated, and the particular subject being treated. Therapy should be initiated at lower doses (in mg/kg/day) in the effective ranges given

above, the dosage thereafter being increased, if necessary, to produce the desired anti-hypertensive effect.

Further, when employed as anti-hypertensive agents or as angiotensin converting enzyme inhibitors, the compounds of the invention may be administered alone or in combination with pharmaceutically acceptable carriers. The proportion and nature of such carriers would be determined by the solubility and other chemical properties of the compound selected, the chosen route of administration, and standard pharmaceutical practice.

The methods of making and using the compounds of the invention are illustrated in the following examples:

## Example 1

## 3-Acetylthiopropanoic Acid t-Butyl Ester

In a 500 ml. Parr hydrogenation bottle chilled in a dry ice-acetone mixture was placed 3-acetylthiopropanoic acid (30g.), methylene chloride (375 ml.), isobutylene (195 ml.), and concentrated suphuric acid (2.0 ml.). The resulting mixture was placed on a mechanical shaker at room temperature for three days. The reaction vessel was chilled with dry ice, and then removed from the shaker. Most of the excess isobutylene was removed by evaporation at room temperature. The residue was added slowly to an excess of powdered sodium carbonate, following by a careful addition of water to the mixture. The pH of the solution was adjusted to 9. The organic layer was collected, washed with saline, then dried over anhydrous magnesium sulphate. Evaporation of the methylene chloride on a rotary evaporator under reduced pressure gave 40 g. of 3-acetylthiopropanoic acid.

Example 2

3 - Mercaptopropanoic Acid t-Butyl Ester

A mixture of 3-acetylthiopropanoic acid t-butyl ester (11.2 g.) and 5.5 N methanolic ammonia (100 ml.) was stored with stirring under nitrogen for 2 hours. The excess reactant (ammonia) and the solvent (methanol) were removed by evaporation on a rotary evaporator under reduced pressure, then kept under vacuum for 1 hour. 3-Mercaptopropanoic acid t-butyl ester thus obtained was used directly in the following reaction.

Example 3

3-[((2S-2- Pyrrolidinylcarbonyl)Thio]Propanoic Acid Trifluoracetate

t-Butoxycarbonyl-L-proline (10.8 g.) was dissolved in dry methylene chloride (400 ml.). 4-Dimethylaminopyridine (0.6 g.) was added to the solution, and the resulting mixture was stirred under nitrogen for 5 minutes. 3-Mercaptopropanoic acid t-butyl ester from the example 2 was dissolved in a small amount of methylene chloride, and the insoluble white solid (acetamide) was removed by suction filtration. The filter residue was washed with methylene chloride. The filtrate and washings were added to the reaction mixture. The stirring reaction mixture thus obtained was chilled to 0° C. by immersing in a dry ice-acetone bath. Dicyclohexylcarbodiimide (10.3 g.) dissolved in a small amount of methylene chloride was added to the reaction mixture, and the chilling source was removed. The temperature of the reaction mixture rose to 5°C in a few minutes. The reaction mixture was kept under stirring at room temperature for 2.5 hours, then was chilled in a dry ice-acetone mixture to - 15°C. Dicyclohexylurea which was separated was removed by filtration. The filter residue

was washed with methylene chloride. The combined filtrate and washing were concentrated on a rotary evaporator under reduced pressure to approximately 150 ml., and washed with 1N cold hydrochloric acid (100 ml.), with saturated aqueous sodium bicarbonate solution (100 ml.), with water, then with saline. It was dried over anhydrous magnesium sulphate overnight and evaporated under reduced pressure on a rotary evaporator, then *in vacuo* to give (2S)-[[[3-(1,1-dimethylethoxy)-3-oxopropyl]thio]carbonyl]-1-pyrrolidinecarboxylic acid (1,1-dimethylethyl)ester as an oil which was amounted to 10.6 g. Cold trifluoroacetic acid (180 ml.) was added slowly under nitrogen to a 1 l. round bottom flask containing the oily product under chilling. The resulting mixture was stirred under chilling in ice for 10 minutes, then at room temperature for 50 minutes. The excess trifluoroacetic acid was evaporated on a rotary evaporator under reduced pressure to dryness. The residue was triturated with anhydrous ether, and evaporated under reduced pressure. The treatment of the residue with anhydrous ether and the evaporation of the ether under reduced pressure was repeated two additional times, then once more with hexanes. The residue was then placed *in vacuo* overnight to give a solid product which weighed 16.6 g. The crude product was washed with anhydrous ether several times. The 3-[((2S)-2-pyrrolidinylcarbonyl)thio] propanoic acid trifluoroacetic acid salt thus obtained melted at 134-136°C.

Analysis for: $C_{10}H_{14}F_3NO_2S$

Calculated: C,37.85; H, 4.45; N, 4.42
Found: C,38.13; H, 4.49; N, 4.45

Example 4

(9aS)-Hexahydro-1H-Pyrrolo[2,1-c][1,4]Thiazepine-1,5-Dione

3-[((2S)-2-Pyrrolidinylcarbonyl)thio] propanoic acid trifluoroacetate (15.3g.) was dissolved in dry methylene chloride. 4-Dimethylaminopyridine (7.3 g.) was added to the solution. The mixture was chilled in a dry ice-acetone bath to -5°. Dicyclohexylcarbodiimide (9.3 g.) dissolved in a small amount of methylene chloride was added to the stirring mixture under nitrogen. The total amount of methylene chloride was 1.5 litres. The temperature of the reaction mixture was raised slowly to 20°C. by immersing in luke warm water, then kept at room temperature. The formation of dicyclohexylurea was noticed at this point. The stirring at room temperature was continued for 4.5 hours. The reaction mixture was concentrated on a rotary evaporator under reduced pressure to approximately 200 ml., and chilled briefly in a dry ice-acetone mixture. A precipitate was collected on a suction filter, and the filter residue was washed with methylene chloride. The combined filtrate and washings were washed successively with cold 1N hydrochloric acid (250 ml. in two times), saturated aqueous sodium carbonate solution (100 ml.), water, and saline, and dried over anhydrous magnesium sulphate. Evaporation of methylene chloride on a rotary evaporator under reduced pressure gave a resinous material which was placed *in vacuo* overnight. The crude product weighed 6.0 g. A portion (3.5 g) of the crude product was purified via a Prep. LC/system 500 (Waters Associates) on a micro porasil column eluted with ethyl acetate. Recrystallization of the oily product obtained with the preparative HPLC from ether gave the crystalline product with melting point 68.5-71°C.

Analysis for: $C_8H_{11}NO_2S$

Calculated C,51.87; H, 5.99; N, 7.56

Found: C, 52.11; H, 6.01; N, 7.49

Example 5

3-Acetylthio-2-methylpropanoic acid t-butyl ester

In a 600 ml. Parr hydrogenation bottle chilled in a dry ice-acetone mixture was placed 3-acetylthio-2-methylpropanoic acid (16.2 g.), methylene chloride (200 ml.), isobutylene (100 ml.) and concentrated sulphuric acid (1 ml). The mixture was placed on a mechanical shaker at room temperature for 3 days. The reaction vessel was chilled with dry ice and removed from the shaker. Most of the excess of isobutylene was removed by distillation at room temperature. The residue was added slowly to an excess of powder sodium carbonate, followed by careful addition of water to the mixture. The pH of the mixture was adjusted to 9. The organic layer was collected, washed with saline, then dried over anhydrous magnesium sulphate. Methylene chloride was evaporated on a rotary evaporator under reduced pressure, and the crude product thus obtained was distilled under vacuum, to give 6.1 g. of 3-acetylthio-2-methylpropanoic acid t-butyl ester, b.p. 73-78$^{C}$ at 0.9 mmHg.

Example 6

3-Mercapto-2-Methylpropanoic Acid t-Butyl Ester

A mixture of 3-acetylthio-2-methylpropanoic acid t-butyl ester (2.18 g.) and 5.5 N methanolic ammonia (20 ml.) was allowed to stir at room temperature for 2 hours. The excess reactant (ammonia) and the solvent (methanol) were removed by evaporation on a rotary evaporator under reduced pressure, then *in vacuo*. The reaction was carried out under nitrogen atmosphere. The product, 3-mercapto-2-methylpropanoic acid t-butyl ester thus obtained was used directly in the following reaction (Example 7).

Example 7

(4S.9aS)-Hexahydro-4-Methyl-1H,5H-Pyrrolo[2,1-c][1,4]-Thiazepine-1.5-Dione

t-Butoxycarbonyl-L-proline (2.15 g.) was dissolved in 25 ml. of dry methylene chloride, and was chilled in an ice bath. 4-Dimethylaminopyridine (70 mg.) was added to the solution, and the resulting solution was stirred under chilling for 10 minutes. 3-Mercapto 2-methylpropanoic acid t-butyl ester from the above reaction (Example 6) was dissolved in dry methylene chloride (10 ml.) Some of acetamide which was also formed in the above reaction (Example 6) could be removed at this point. Acetamide is less soluble in methylene chloride than 3-mercapto-2-methylpropanoic acid, and thus the insoluble acetamide was separated by filtration. The methylene chloride filtrate was added to the reaction mixture of t-butoxycarbonyl-L-proline and 4-dimethylaminopyridine. Addition of dicyclohexylcarbodiimide (2.1. g.) dissolved in a minimum amount of methylene chloride was followed. The resulting mixture was stirred under chilling in ice for 10 minutes. Then the ice bath was removed, and the stirring was continued at room temperature for 3 hours. The mixture was chilled in ice, and precipitate (dicyclohexylurea) was removed by filtration and washed with methylene chloride. The combined filtrate and washings were evaporated under reduced pressure, then _in vacuo_. The residue was dissolved in about 20 ml. of methylene chloride, and any insoluble material was removed by filtration. The filtrate was diluted to make a total volume of about 60 ml. and was washed successively with cold 1N hydrochloric acid (50 ml.in two times) saturated aqueous sodium carbonate solution, water and saline, and then dried over anhydrous magnesium sulphate. Evaporation of the methylene chloride on a rotary evaporator under reduced pressure gave an oil which amounted to 4.2 g. The residue was dissolved in ether (an ether insoluble material at this point was

removed by filtration), and the ether solution was evaporated on a rotary evaporator under reduced pressure, then *in vacuo* to give (2S)-[[[3-(1,1-dimethylethoxy)-2-methyl-3-oxopropyl]thio]carbonyl]-1-pyrrolidinecarboxylic acid (1,1-dimethylethyl)ester as an oil.

The oil (2.9 g.) thus obtained was stirred gently with trifluoroacetic acid (25 ml.) for 1 hour, then evaporated under reduced pressure on a rotary evaporator to give an oily residue. The residue was dissolved in a small amount of anhydrous ether, and the solution was evaporator under reduced pressure. The dissolution of the residue followed by evaporation was repeated two additional times with ether then once with hexane. The oily product which subsequently solidified was dried under vacuo overnight in a drying pistol over phosphorus pentoxide and potassium hydroxide.

The dried product (2.1 g.) was dissolved in 200 ml. of methylene chloride and chilled in an ice bath. 4-Dimethylaminopyridine (1.16 g.) was added, and the mixture was stirred under chilling in ice for 5 minutes. Dicyclohexylcarbodiimide (1.31 g.) dissolved in a minimum amount of methylene chloride was added to the reaction mixture. An additional 150 ml. of methylene chloride was added. The resulting mixture was allowed to stir with chilling in ice for 15 minutes, then at room temperature for 5 1/4 hours, and kept in a refrigerator for several hours. The reaction mixture was concentrated on a rotary evaporator under reduced pressure to approximately 100 ml. and chilled in ice. A precipitate was removed by suction filtration and washed with methylene chloride. The combined filtrate and washings was washed successively with cold 1N hydrochloric acid (150 ml. in two times), saturated aqueous sodium bicarbonate solution, water, and saline, then dried over anhydrous magnesium sulphate. Evaporation of the solvent under reduced pressure on a rotary evaporator, then *in vacuo* gave an oily residue. The crude product was purified

*via* a Prep LC/System 500 (Waters Associates) on a micro porasil column eluted with a solution of methylene chloride (90 by vol.) and ethyl acetate (10 by vol.). Fractions No.7. - No. 10 were combined and evaporated under reduced pressure to give a solid residue. Recrystallization of the residue from ether gave (4S, 9aS)-hexahydro-4-methyl-1H,5H-pyrrolo[2,1-c][1,4]thiazepine-15-dione (200 mg.) which melted at 102-103.5°C, $[\alpha]_D^{24}$= -113.6 (0.835%, EtOH).

This material is identical with (4S, 9aS) hexahydro-4-methyl-1 H, 5 H-pyrrolo[2,1-c][1,4] thiazepine-1, 5-dione prepared from 1-(D-3-mercapto-2-methyl-propanoyl)-L-proline (S,S) described in Example 8. The mixture melting point of both compounds was not depressed, and the infrared and mass spectra of the two compounds were identical. The experiments described in this example were carried out under nitrogen atmosphere.

## Example 8

## (4S,9aS)-Hexahydro-4-methyl- 1 H -pyrrolo[2,1-c] [1,4]-thiazepine-1,5-dione

1-(D-3-Mercapto-2-methylpropanoyl)-L-proline (S,S) (0.65 g.) was dissolved in methylene chloride (150 ml.) and the resulting solution was chilled in ice. 4-Dimethyl-aminopyridine (0.37 g.) was added and the mixture was stirred vigorously for 5 minutes. Dicyclohexylcarbodiimide (0.62 g.) was added to the mixture. The reaction mixture thus obtained was stirred for 15 minutes in an ice bath, then the ice bath was lowered, and the stirring was continued for 3 hours. The reaction mixture was then concentrated *in vacuo* to about 60 ml. by the use of a rotary evaporator. The concentrated solution was chilled in ice, and a precipitate was removed by filtration under suction. The filter residue was washed with methylene chloride. The combined filtrate and washings were washed with approximately 1N hydrochloric acid twice, with saturated aqueous sodium bicarbonate

solution, with saline, and then dried over anhydrous magnesium sulphate. Evaporation of methylene chloride under reduced pressure on a rotary evaporator gave a solid residue which was extracted with ether. The ether extract was evaporated under reduced pressure to give a solid residue which was purified by a preparative HPLC on a micro porasil column using ethyl acetate as the eluent. The product (160 mg.) melted at 102-103.5°C. The melting point was depressed when taken admixed with the starting material, 1-(D-3-mercapto-2-methylpropanoyl)-L-proline (S,S).

<u>Analysis for</u>: $C_9H_{13}NO_2S$
<u>Calculated</u>: C, 54.25; H, 6.57; N, 7.03
<u>Found</u>: C, 54.06; H, 6.56; N, 6.94

## Example 9

**(4R,9aS)-Hexahydro-4-Methyl-1 H, 5H-Pyrrolo[2,1-c][1,4]-Thiazepine-1, 5-Dione**

Fractions No. 15 - No. 18 of the preparative HPLC in Example 7 were combined, and evaporated under reduced pressure to give a solid residue which was then recrystallized from anhydrous ether to give the title compound (207 mg.), m.p. 74-76°C.

$[\alpha]_D^{24} = -36.02$ (0.66%, EtOH).

<u>Analysis for</u>: $C_9H_{13}NO_2S$
<u>Calculated</u>: C, 54.24; H, 6.57; N, 7.03
<u>Found</u>: C,54.27; H, 6.69; N, 7.19

Example 10

(8aS)-Tetrahydro-1H-Pyrrolo[2,1-c][1,4]Thiazine-1,4(3H)-Dione

1-(2-Mercaptoacetyl)-L-proline (2.1g.) was dissolved in 500 ml. of methylene chloride, and the resulting solution was chilled under nitrogen to 15° in dry ice-acetone mixture. 4-Dimethylaminopyridine (1.3 g.) was added, and the mixture was stirred for 5 minutes. Dicyclohexylcarbodiimide (2.18 g.) which was dissolved in a small amount of methylene chloride was added to the mixture with stirring. The chilling source was removed in 15 minutes, and the stirring was continued at room temperature overnight. The reaction mixture was evaporated on a rotary evaporator under reduced pressure to about 100 ml. A precipitate was removed by filtration, and the filter residue was washed with methylene chloride several times. The combined filtrate and washings were washed successively with 1N hydrochlorid acid, saturated aqueous sodium bicarbonate solution, water and saline, then dried over magnesium sulphate. Evaporation of methylene chloride on a rotary evaporator under reduced pressure gave a white solid. The crude product was recrystallized from ether to give 0.74 g. of the titled compound, m.p. 116-118°. Another recrystallization from ether improved the m.p. to 116.5-118°C.

Analysis for: $C_7H_9NO_2S$

Calculated: C,49.10; H, 5.29; N, 8.18

Found: C,48.99; H, 5.21; N 8.15

## EXAMPLE 11

2-Methyl-3-[(2S)-Pyrrolidinylcarbonyl)Thio]Propanoic Acid, Trifluoroacetic Acid Salt

(2S)-2-[[[3-(t-Butoxy)-2-methyl-3-oxopropyl]thio] carbonyl]-1-pyrrolidine carboxylic acid t-butyl ester (69 g.) was magnetically stirred under a nitrogen atmosphere and chilled to -5$^{o}$ with a dry ice-acetone bath. Cold trifluoroacetic acid (900 ml.) was added slowly at first and then more rapidly over 30 min. The reaction solution was warmed to 23$^{o}$ and stirred at room temperature for one hour. Evaporation on the rotary evaporator (no heat) afforded an oily residue which was vigorously shaken with anhydrous ether and evaporated on a rotary evaporator. The ether co-evaporation was repeated; and, then the residue was slurried in hexane and decanted. The hexane wash was also repeated. The residue was shaken vigorously with hexane, and the hexane was evaporated on a rotary evaporator under reduced pressure. The residue was then placed under high vacuum overnight. The residue was triturated with 300 ml. of anhydrous ether at 10$^{o}$, seeded, and vigorously stirred for 1/2 to 1 hour to induce crystallization. Filtration of the solid followed by washing with cold ether gave the above named product (42.0 g.), m.p. 81-83$^{o}$.

Analysis for: $C_9H_{15}NO_3S \cdot CF_3COOH$

Calculated: C, 39.67; H, 4.87; N, 4.23

Found: C, 40.13; H, 4.84; N, 4.18.

## EXAMPLE 12

(4S,9aS)-Hexahydro-4-Methyl-1H,5H-Pyrrolo[2,1-c][1,4]-Thiazepine-1,5-Dione

2-Methyl-3-[(2S)-pyrrolidinylcarbonyl)thio]propanoic acid, trifluoroacetic acid salt (2.1 g.) was dissolved in 200 ml. of methylene chloride and chilled in an ice bath.

4-Dimethylaminopyridine (1.16 g.) was added, and the mixture was stirred under chilling in ice for 5 minutes. Dicyclohexylcarbodiimide (1.31 g.) dissolved in a minimum amount of methylene chloride was added to the reaction mixture. An additional 130 ml. of methylene chloride was added. The resulting mixture was allowed to stir with chilling in ice for 15 minutes, then at room temperature for 5 1/4 hours, and kept in a refrigerator for several hours. The reaction mixture was concentrated on a rotary evaporator under reduced pressure to approximately 100 ml. and chilled in ice. A precipitate was removed by suction filtration and washed with methylene chloride. The combined filtrate and washings was washed successively with cold 1N hydrochloric acid (150 ml. in two times), saturated aqueous sodium bicarbonate solution, water, saline, then dried over anhydrous magnesium sulphate. Evaporation of the solvent under reduced pressure on a rotary evaporator, then *in vacuo* gave an oily residue. The crude product was purified *via* a Prep LC/System 500 (Waters Associates) on a micro porasil column eluted with a solution of methylene chloride (90 by vol.) and ethyl acetate (10 by vol.). Fractions No. 7-No. 10 were combined and evaporated under reduced pressure to give a solid residue. Recrystallisation of the residue from ether gave (4S,9aS)-hexahydro-4-methyl-1*H*,5*H*-pyrrolo[2,1-*c*][1,4]thiazepine-1,5-dione (200 mg.) which melted at 102-103.5°C $[\alpha]_D^{24}$= 113.6 (0.835% EtOH). [Starting material from Example 11].

Analysis for: $C_9H_{13}NO_2S$

Calculated: C, 54.25, H, 6.57 N, 7.03

Found: C, 54.30, H, 6.64; N, 7.10.

EXAMPLE 13

Mercaptoacetic Acid t-Butyl Ester

A mixture of acetyl mercaptoacetic acid t-butyl ester (9.5 g.) and 5.5 N methanolic ammonia (100 ml.) was stirred gently under nitrogen for 2 hours. The excess reactant (ammonia) and the solvent (methanol) were removed by evaporation on a rotary evaporator under reduced pressure, and the residue was kept under vacuum for 1 hour. The product thus obtained was used directly in the following reaction (Example 14).

EXAMPLE 14

2-[((2S)-2-Pyrrolidinylcarbonyl)Thio]-Acetic Acid Trifluoroacetate

t-Butoxycarbonyl-L-proline (10.76 g.) was dissolved in dry methylene chloride (300 ml.). 4-Dimethylaminopyridine (0.6 g.) was added to the solution, and the resulting mixture was stirred under nitrogen for 5 minutes. Dicyclohexylcarbodiimide (10.3 g.) dissolved in a small amount of methylene chloride (35 ml.) was added to the mixture. Addition of mercaptoacetic acid t-butyl ester from Example 13 was followed immediately. The resulting mixture was stirred for 20 minutes under chilling in an ice bath, then 4 hours at room temperature. The reaction mixture was chilled in dry ice-acetone mixture for 0.5 hours. The precipitate which separated was collected on a filter and washed with methylene chloride. The combined filtrate and washings were concentrated on a rotary evaporator under reduced pressure to approximately 150 ml. The concentrated solution was washed with cold 1N hydrochloric acid (2 times), with saturated aq. sodium bicarbonate solution, then with saline (2 times), and dried over magnesium sulphate. Evaporation of the solvent on a rotary evaporator under reduced pressure, then *in vacuo* gave 19 g. of (2,S)-[[[3-(1,1-dimethylethoxy)-3-oxoethyl] thio]carbonyl]-1-pyrrolidinecarboxylic acid (1,1-dimethoxyethyl)

ester as an oil.

Chilled trifluoroacetic acid (200 ml.) was added slowly to the above residue under nitrogen while chilling in ice. The addition of trifluoroacetic acid required about 20 minutes. The reaction mixture thus obtained was stirred gently at room temperature for 1 hour. Evaporation of the trifluoroacetic acid on a rotary evaporator afforded an oily residue. The residue was vigorously shaken with anhydrous ether, then evaporated on a rotary evaporator. The ether coevaporation was repeated two more times. The residue was then slurried in hexane and decanted. The hexane wash was repeated. The residue was shaken vigorously with a mixture of hexane and ether (3:1), and evaporated on a rotary evaporator under reduced pressure, and the residue was kept under high vacuum to give the title product (17.8 g.).

EXAMPLE 15

(8aS)-Tetrahydro-1*H*-Pyrrolo[2,1-*c*]-[1,4]Thiazine-1,4-(3*H*)-Dione

2-[((2S)-2-Pyrrolidinylcarbonyl)thio]acetic acid trifluoroacetate (17.8 g. from Example 14) was dissolved in methylene chloride (1.1), and the solution was chilled in ice. To the solution was added under stirring 4-dimethylaminopyridine until the solution became neutral. It required approximately 9 g. In about 5 minutes, dicyclohexylcarbodiimide (12.11 g.) dissolved in methylene chloride (20 ml.) was added slowly to the mixture. The resulting mixture was kept under stirring in ice for 15 minutes, then for 4.5 hours at room temperature. The reaction mixture was chilled in dry ice-acetone mixture for 20 minutes, and a precipitate was removed by filtration, and washed with methylene chloride. The combined filtrate and washings were concentrated on a rotary evaporator to about 250 ml. The concentrated solution was washed with cold 1N hydrochloric acid 3 times, with cold water, then with saline, and dried over anhydrous magnesium sulphate.

Evaporation of the solvent on a rotary evaporator under reduced pressure gave an oily residue which was kept in vacuo. The residue was dissolved in anhydrous ether (there was a considerable amount of insoluble material which was removed by filtration). The ether solution was concentrated on a rotary evaporator to approximately 20 ml. Chilling of the concentrated solution in a freezer caused separation of a precipitate which was collected on a filter and washed with ether. The product melted at 115-117°, and weighed 1.5 g. Mixture melting point with (8aS)-tetrahydro-1H-pyrrolo[2,1-c][1,4]thiazine-1,4(3H)-dione prepared from 1-(2-mercaptoacetyl)-L-proline (Example 10) was not depressed.

## EXAMPLE 16

(4S,9aS)-Hexahydro-4-Methyl-1H,5H-Pyrrolo[2,1-c][1,4]-Thiazepine-1,5-Dione

Add the acid chloride hydrochloride (0.01 mole) derived from 2-methyl-3-[(2S)-pyrrolidinylcarbonyl)thio] propanoic acid, trifluoroacetic acid salt of Example 11 in small portions with stirring to an ice-cooled solution of triethylamine (0.02 mole) in methylene chloride (20 ml.). Continue stirring the cooled solution for 6 hours. Recover the title compound from the reaction mixture in a similar manner to Example 12.

## EXAMPLE 17

(8aS)-Tetrahydro-1H-Pyrrolo[2,1-c]-[1,4]Thiazine-1,4-(3H)-Dione

Add the acid chloride from 1-bromoacetyl-L-proline in small portions to an ice-cooled solution of potassium hydrogen sulphide (0.02 mole) in ethanol (10 ml.) with stirring. Continue stirring the chilled mixture for 1 hour. Separate the precipitated potassium chloride. Heat the reaction mixture on a water bath for 3 hours. Remove the precipitated potassium bromide and ethanol,

wash the product with water and dry over magnesium sulphate. Recrystallisation from ether gives the title compound.

CLAIMS

1. A compound of the formula:

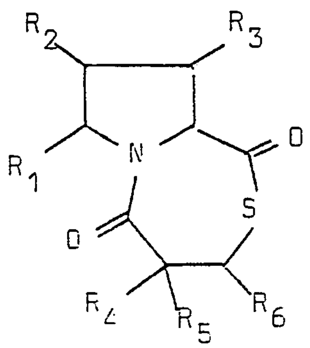
(Ia)

or

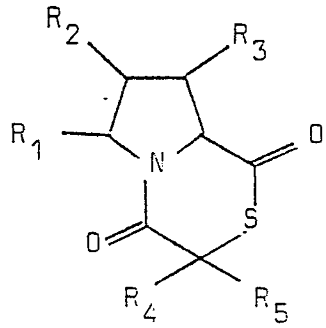
(Ib)

in which $R_1$ is hydrogen or lower alkyl; $R_2$ and $R_3$ are, independently, hydrogen, hydroxy, halogen, lower alkyl, lower alkoxy, or aryl and $R_4$, $R_5$ and $R_6$ are, independently, hydrogen, lower alkyl, lower hydroxyalkyl, aryl, or arylalkyl, except that $R_4$ and $R_5$ may not both be aryl.

2. A compound as claimed in Claim 1, in which (a) $R_2$ and $R_3$ are hydrogen, or (b) $R_1$, $R_5$ and, where present, $R_6$ are hydrogen.

3. A compound as claimed in Claim 1, in which (a) $R_1$, $R_2$ and $R_3$ are hydrogen or (b) $R_2$, $R_3$, $R_5$ and, where present, $R_6$ are hydrogen.

4. A compound as claimed in Claim 1, in which $R_1$, $R_2$, $R_3$, $R_5$ and, there present, $R_6$ are hydrogen.

5. A compound as claimed in Claim 4, in which $R_4$ is hydrogen or lower alkyl.

6. A compound as claimed in Claim 1 in which $R_1$, $R_2$, $R_3$ and $R_5$ are hydrogen and $R_4$ and, where present, $R_6$ are hydrogen or lower alkyl.

7. A compound as claimed in Claim 5, which is (a) the 4S, 9aS, or 3S, 8aS stereoisomer or (b) the 4R, 9aS or 3R, 8aS stereoisomer.

8. A compound as claimed in any one of Claims 1 to 7, which has formula Ia.

9. A compound as claimed in any one of Claims 1 to 7, which has formula Ib.

10. A compound selected from (4S,9aS)-hexahydro-4-methyl-1H,5H-pyrrolo[2,1-c][1,4]thiazepine-1,5-dione; (4R,9aS)-hexahydro-4-methyl-1H,5H-pyrrolo[2,1-c]-[1,4]thiazepine-1,5-dione; 9aS)-hexahydro-1H,5H-pyrrolo[2,1-c][1,4]thiazepine-1,5-dione; 4-ethyl-(4S,9aS)-hexahydro-1H,5H-pyrrolo[2,1-c][1,4]-thiazepine-1,5-dione; (8aS)-tetrahydro-1H-pyrrolo-[2,1-c][1,4]-thiazine-1,4-dione; (3S,8aS)-tetrahydro-3-methyl-1H-pyrrolo[2,1-c][1,4]thiazine-1,4-(3H)-dione; and (3R,8aS)-tetrahydro-3-methyl-1H-pyrrolo [2,1-c][1,4]thiazine-1,4-(3H)-dione.

11. As a pharmaceutical, a compound as claimed in any one of Claims 1 to 10.

12. A pharmaceutical composition comprising a compound as claimed in any one of Claims 1 to 10 and a pharmaceutically acceptable carrier.

13. A process for the preparation of a compound having the general formula

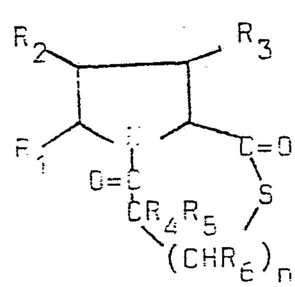

(I)

(wherein $R_1$, $R_2$, $R_3$, $R_4$, $R_5$, $R_6$ are as defined in Claim 1 and n is 1 or 0), which comprises

(A) cyclising a compound having the general formula

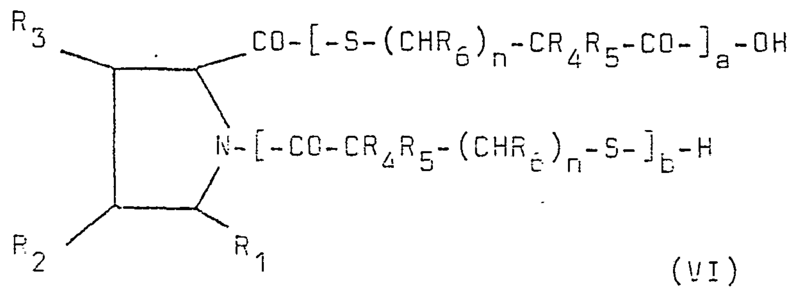

(VI)

(wherein $R_1$, $R_2$, $R_3$, $R_4$, $R_5$, $R_6$ and n are as defined above, one of a and b represents 0 and the other one of a and b represents 1) or a reactive derivative thereof; or

(B) cyclising a compound having the formula

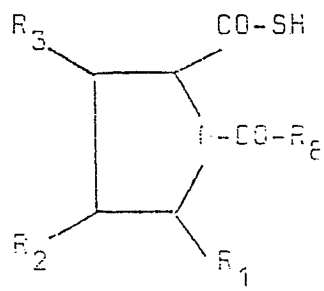

(VII)

(wherein $R_1$, $R_2$ and $R_3$ are as defined above subject to the proviso that neither of $R_2$ and $R_3$ is halogen and $R_8$ represents a group having the formula $-CR_4R_5-(CHR_6)_n-R_7$ or $-CR_4=CHR_6$ where $R_4$, $R_5$, $R_6$ and n are as defined above, subject to the proviso that none of $R_4$, $R_5$ and $R_6$ is halophenyl, and $R_7$ is a leaving group or atom) or a salt thereof.

4. A compound having the general formula

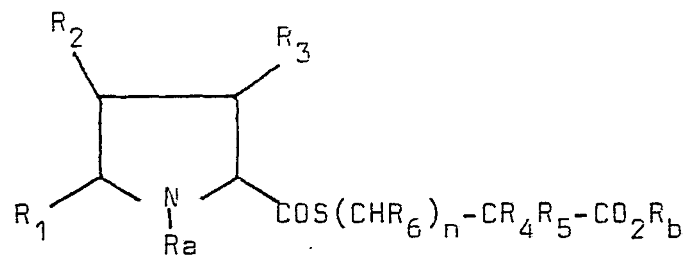

(II)

in which $R_1$ is hydrogen or lower alkyl; $R_2$ and $R_3$ are, independently, hydrogen, hydroxy, halogen, lower alkyl, lower alkoxy, or aryl; $R_4$, $R_5$ and $R_6$ are, independently, hydrogen, lower alkyl, lower hydroxyalkyl, aryl, or arylalkyl, except that $R_4$ and $R_5$ may not both be aryl; n is 1 or 0; $R_a$ is hydrogen or an amino protecting group, and $R_b$ is hydrogen, a carboxylic acid protecting group or a group having the formula

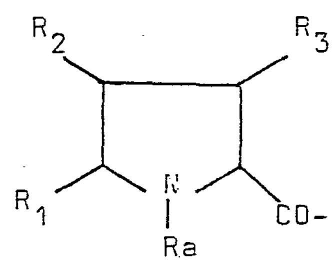

(III)

(wherein $R_a$, $R_1$, $R_2$ and $R_3$ are as defined above) or a salt thereof.

15. A compound which is 2-methyl-3-[(2-pyrrolidinylcarbonyl)thio]propanoic acid or a salt thereof, or 3-[(2-pyrrolidinylcarbonyl)thio]propanoic acid or a salt thereof.

## CLAIMS FOR AUSTRIA

1. A process for the preparation of a compound of the formula:

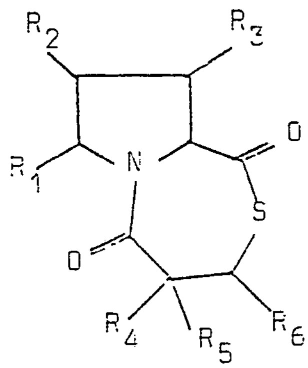

(Ia)

or

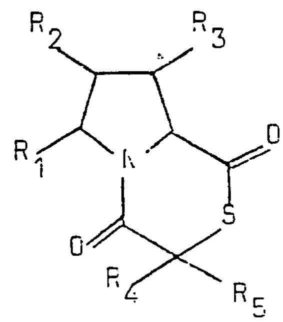

(Ib)

in which $R_1$ is hydrogen or lower alkyl; $R_2$ and $R_3$ are, independently, hydrogen, hydroxy, halogen, lower alkyl, lower alkoxy, or aryl and $R_4$, $R_5$ and $R_6$ are, independently, hydrogen, lower alkyl, lower hydroxyalkyl, aryl, or arylalkyl, except that $R_4$ and $R_5$ may not both be aryl, which comprises

(A) cyclising a compound having the general formula

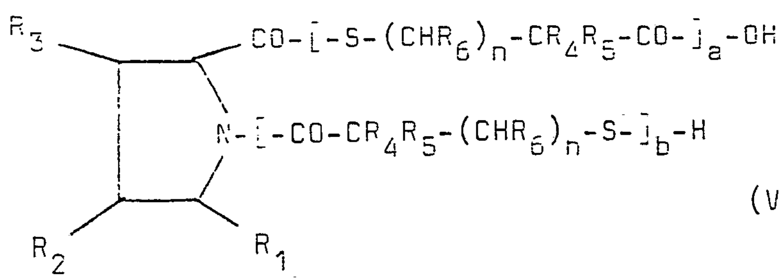

(VI)

(wherein $R_1$, $R_2$, $R_3$, $R_4$, $R_5$, $R_6$ are as defined above and n is 1 or 0, one of a and b represents 0 and the other one of a and b represents 1) or a reactive derivative thereof; or

(B) cyclising a compound having the formula

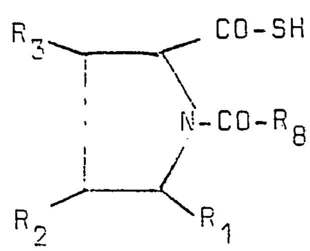

(VII)

(wherein $R_1$, $R_2$ and $R_3$ are as defined above subject to the proviso that neither of $R_2$ and $R_3$ is halogen and $R_8$ represents a group having the formula $-CR_4R_5-(CHR_6)_n-R_7$ or $-CR_4=CHR_6$ where $R_4$, $R_5$, $R_6$ and n are as defined above, subject to the proviso that none of $R_4$, $R_5$ and $R_6$ is halophenyl, and $R_7$ is a leaving group or atom) or a salt thereof.

2. A process as claimed in Claim 1, which comprises cyclising a compound having the formula VI with a coupling reagent

3. A process as claimed in Claim 2, wherein the coupling reagent is dicyclohexylcarboxiimide.

4. A process as claimed in Claim 1 or 2 wherein 4-dimethylaminopyridine is present as a catalyst.

5. A process as claimed in Claim 1, which comprises cyclisation of a compound having formula VII or a salt thereof.

6. A process as claimed in any one of Claims 1 to 5 in which (a) $R_2$ and $R_3$ are hydrogen or (b) $R_1$, $R_5$ and, where present, $R_6$ are hydrogen.

7. A process as claimed in any one of Claims 1 to 5, in which (a) $R_1$, $R_2$ and $R_3$ are hydrogen or (b) $R_2$, $R_3$, $R_5$ and, where present, $R_6$ are hydrogen.

8. A process as claimed in any one of Claims 1 to 5, in which $R_1$, $R_2$, $R_3$, $R_5$ and, where present, $R_6$ are hydrogen and $R_4$ is hydrogen or lower alkyl.

9. A process as claimed in Claim 8, wherein the compound having formula Ia or Ib is (a) the 4S, 9aS, or 3S, 8aS stereoisomer or (b) the 4R, 9aS or 3R, 8aS stereoisomer.

10. A process for the preparation of a compound having the general formula

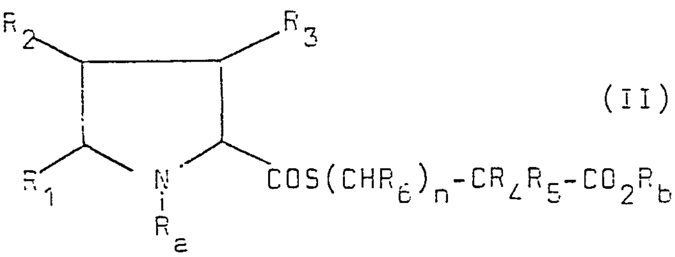

(II)

in which $R_1$ is hydrogen or lower alkyl; $R_2$ and $R_3$ are, independently, hydrogen, hydroxy, halogen, lower alkyl, lower alkoxy or aryl; $R_4$, $R_5$ and $R_6$ are, independently, hydrogen, lower alkyl, lower hydroxyalkyl, aryl or arylalkyl, except that $R_4$ and $R_5$ may not both be aryl; n is 1 or 0; $R_a$ is hydrogen or an amino protecting group, and $R_b$ is hydrogen, a carboxylic acid protecting group or a group having the formula

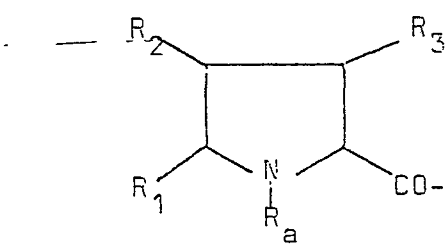

(III)

(wherein $R_a$, $R_1$, $R_2$ and $R_3$ are as defined above) or a salt thereof, which comprises

(A) acylation of a compound having the general formula

$$HS-(CHR_6)_n-CR_4R_5-CO_2H \quad (XI)$$

(wherein $R_4$, $R_5$, $R_6$ and n are as defined above) or a derivative thereof with an acid having the general formula

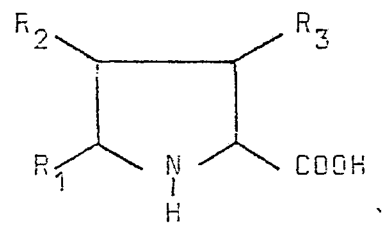

(XII)

(wherein $R_1$, $R_2$ and $R_3$ are as defined above) or a derivative thereof to form a thioester, or

(E) reaction of a thioacid having the formula

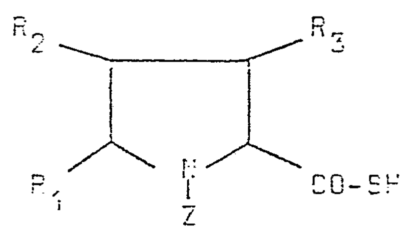

(XV)

(wherein Z is an amino protecting group, $R_1$, $R_2$ and $R_3$ are as defined above subject to the proviso that neither of $R_2$ and $R_3$ is halogen) or a salt thereof with a compound having the formula $R_8-CO_2R_b$ (wherein $R_b$ is as defined above and $R_8$ is a group having the formula $-CR_4R_5-(CHR_6)_n-R_7$ or $-CR_4=CHR_6$ where $R_4$, $R_5$, $R_6$ and n are as defined above subject to the proviso that none of $R_4$, $R_5$ and $R_6$ is halo-phenyl, and $R_7$ is a leaving group or atom,

and, if desired

(a) removal of a crboxylic acid protecting group and/or a secondary amino protecting group; or

(b) hydrolysis of a compound where $R_b$ has formula III to form an acylmercapto carboxylic acid or

(c) conversion of a compound having formula II into a salt thereof or a salt into a compound having formula II.

BAD ORIGINAL